(19) Europäisches Patentamt
European Patent Office
Office européen des brevets

(11) **EP 3 345 612 B1**

(12) **EUROPEAN PATENT SPECIFICATION**

(45) Date of publication and mention of the grant of the patent:
**15.12.2021 Bulletin 2021/50**

(21) Application number: **15903147.5**

(22) Date of filing: **03.09.2015**

(51) Int Cl.:
*A61K 38/02* (2006.01)     *A61K 35/74* (2015.01)
*A61K 8/99* (2017.01)      *A61P 17/06* (2006.01)
*A61P 17/04* (2006.01)     *A61Q 19/00* (2006.01)

(86) International application number:
**PCT/RU2015/000560**

(87) International publication number:
**WO 2017/039476 (09.03.2017 Gazette 2017/10)**

(54) **USE OF BACTERIORHODOPSIN AS A COSMETIC AND/OR THERAPEUTIC AGENT IN THE COMPREHENSIVE TREATMENT OF DERMATOSES**

VERWENDUNG VON BAKTERIORHODOPSIN ALS KOSMETIKUM UND/ODER THERAPEUTIKUM ZUR UMFASSENDEN BEHANDLUNG VON DERMATOSEN

UTILISATION DE BACTÉRIORHODOPSINE EN TANT QUE PRODUIT COSMÉTIQUE ET/OU MÉDICAMENTEUX LORS D'UN TRAITEMENT COMPLEXE DE DERMATOSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.07.2018 Bulletin 2018/28**

(73) Proprietors:
• **Grishkova, Elizaveta Sergeevna**
  **Moscow 117405 (RU)**
• **Shnyra, Alexandre**
  **Overland Park, KS 66213 (US)**

(72) Inventor: **GRISHKOVA, Elizaveta Sergeevna**
**Moscow, 117405 (RU)**

(74) Representative: **Bucher, Ralf Christian**
**Patentanwalt Dipl.-Ing.**
**Alte Landstrasse 23**
**85521 Ottobrunn (DE)**

(56) References cited:
**WO-A1-2016/028188     WO-A2-01/23415**
**RU-C1- 2 109 515      RU-C1- 2 370 957**
**RU-C1- 2 558 237      RU-C2- 2 303 977**
**US-A1- 2014 134 701**

• **None**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The invention relates to the field of medicine, in particular to cosmetology, and concerns the development of therapeutic and cosmetic products used in cosmetology and dermatology.

Background of the invention

In modern cosmetology, three main branches are distinguished: aesthetic, medical and treatment-and-prophylactic cosmetology. Aesthetic cosmetology deals with a wide range of issues related to maintaining the normal functioning of the skin and its appendages; medical cosmetology treats skin and its appendages at the medical level; and the treatment-and-prophylactic cosmetology, the so-called cosmeceuticals, is aimed at eliminating and preventing dermatological disorders and dehydration of the skin, slowing down its aging processes; it includes active components of predominantly natural origin, which can suppress inflammatory processes, remove free radicals and reduce their formation, improve blood circulation, influence metabolic and regenerative processes in the skin.

To date, cosmeceuticals are of great interest due to the fact that cosmetics of this class work at the cellular level, i.e. they can have a positive effect on the biochemical processes in the body. The composition of such cosmetics is non-toxic and safe for the skin and the body as a whole, as it contains natural bioactive components obtained through high technologies and scientific development in the field of nutritiology (cell nutrition). The production is used by people suffering from diseases such as psoriasis, eczema, etc., since it is 94-98% hypoallergenic. Cosmeceuticals have a prolonged positive effect on the skin, i.e. the result achieved by rejuvenating the skin is maintained for a long time after the abolition of these products, since they work in five layers of the epidermis and two layers of the dermis to the hypodermis.

Cosmeceutical agents include preparations containing, for example:

- Fruit acids (AHA) - soft peelings smoothing the skin;

- Vitamin E - a complex antioxidant, a "trap" for free radicals that has an antiinflammatory effect and protects the skin from premature aging, preventing the formation of wrinkles;

- Vitamin D - has a noticeable activity in the areas of cell proliferation and differentiation;

- Vitamin A and sea buckthorn oil - promote the acceleration of epithelialization;

- Hyaluronic acid - a natural moisturizer that protects the skin from drying out;

- Ceramides - components that act as a barrier in the stratum corneum and serve to restore the natural state of the skin;

- Sun protection factors - UV filters preventing skin burns by UV-rays.

- Medicinal plants such as Matricaria, Rose, Aloe, Panax, Hypericum, Urtica, Parsley, Sorbus, Cucumber - suppress the growth of pathogenic microorganisms;

[0005] - A suspension of biologically active components of bifidobacteria and halobacteria - stimulates metabolic processes in the skin, promotes the regeneration of epidermal cells.

From the point of view of production and obtaining of cosmetic products for skin care technology, the suspensions of biologically active components of bifidobacteria and halobacteria are the most attractive. The seasonality factor is excluded in the process of obtaining these suspensions, in contradistinction to the case with medicinal plants. In addition, the suspensions themselves already contain biologically active compounds synthesized by microorganisms during their growth. All these biologically active substances were synthesized and balanced by nature itself; therefore, they are not alien to the body and are perfectly assimilated. A disadvantage of such suspensions is a limited range of application; more precisely, they are used only in the complex treatment of skin problems.

From the state of the art, it is also known to use a suspension of active components based on halobacterial biomass. Based on the strain of halophilic bacteria Halobacterium halobium 353 Pushchinsky, a preparation was produced that is a lyophilically dried powder of halobacterial biomass of the non-pathogenic strain Halobacterium Halobium 353P called "Baxin", disclosed in RU 2109515 patent published on 27.04.1998. The "Baxin" powder is used as a biologically active additive to food - nutraceutical.

Summary of the invention

The task for solving by the claimed invention is to expand the arsenal of cosmetic products used as cosmetic skin care products in the complex treatment of dermatoses such as psoriasis, eczema, neurodermatitis, and to maintain the normal functioning of the facial and body skin.

The technical result achieved in the implementation of the claimed invention consists in the creation of a new preparation possessing the properties of a medical-cosmetic agent, by using bacteriorhodopsin as an active component of a cosmetic skin care product in the norm and/or a therapeutic agent in the complex treatment of dermatoses by disclosing new specific biological features of bacteriorhodopsin of halophilic bacteria Halobacterium salinarum VKPM V-11850.

This technical result is achieved by using an aqueous solution of bacteriorhodopsin of the strain of halophilic bacteria Halobacterium salinarum VKPM V-11850 containing bacteriorhodopsin at a concentration of 0.24%-0.75% as a cosmetic agent for maintaining the normal functioning of the facial and body skin and/or a remedy for complex treatment of dermatoses.

Bacteriorhodopsin is a retinal-containing protein of purple membranes of Halobacterium salinarum. Retinal is a derivative of vitamin A.

Strains of halophilic bacteria Halobacterium salinarum are natural inhabitants of salt lakes, where the concentration of NaCl can reach 4M, which is 6 times higher than in seawater. Bacteriorhodopsin was previously used as a plant growth stimulant and, in this case, bacterinhodopsin retinal was used as a physiological regulator of vital activity of plant cells. However, as it turned out during the research, bacteriorhodopsin favorably affects not only the growth and development of plant cells, but also the growth and normal functioning of human skin cells. This allows the use of bacteriorhodopsin for a new purpose, namely, to obtain pharmaceuticals that have the necessary properties to maintain the normal functioning of the facial and body skin or in the complex treatment of skin problems.

Bacteria of the Halobacterium salinarum genus do not possess zoopathogenic or phytopathogenic properties and do not pose danger to humans, so working with them does not require special precautions. These findings are confirmed by the research of Thomas Cavalier-Smith, published in his article "Cavalier-Smith T. The neomuran origin of archaebacteria, the negibacterial root of the universal tree and bacterial megaclassification. Int. J. of Syst. And Envir.Microb. 2002, 52, 7-76".

Embodiments of the Invention

The bacteriorhodopsin preparation is obtained as follows.

The strain of halophilic bacteria Halobacterium salinarum VKPM V-11850 is grown in 750 ml flasks in a volume of a growing medium of 300 ml under fluorescent lighting until the stationary growth stage on a circular shaker at 37°C and shaking 100 rpm. For cultivation, the medium of the following composition is used (mass%): peptone — 1, yeast extract — 0.5, NaCl — 25, MgSO$_4$ — 2, KCl — 0.2, sodium citrate — 0.3, glycerin — 0.1, CaCl$_2$ — 0.02, water - the rest, pH of the environment — 7.2-7.4.

After 6 days, the precipitate is separated by centrifugation at 7000 g for 10 minutes and the obtained biomass is weighed. Subsequently, distilled water in a volume of 300 ml is added to the precipitate (20 g) and incubated with stirring at room temperature for 16 hours.

After centrifugation at 7000 g for 10 min and 4°C, the precipitate is discarded and the resulting supernatant is centrifuged at 50,000 g for 40 min and 4°C. The supernatant is discarded, distilled water is poured into the resulting precipitate in the same volume, the precipitate is suspended, and the resulting bacterial lysate suspension is centrifuged at 50,000 g for 40 minutes and 4°C. Such manipulation of suspending the precipitate in water followed by high-speed centrifugation is repeated until the ratio of the optical density of the solution at 280 nm to the optical density of the solution at a wavelength of 570 nm (D280\D570) is less than 2.5 in the suspended precipitate after the next centrifugation. After that, the supernatant is diluted with distilled water to a concentration of bacteriorhodopsin of 2.4 mg/ml, 7.2 mg/ml. The amount of bacteriorhodopsin is determined at a wavelength of 570 nm, and the concentration is calculated by the following formula:

$$C = D \cdot Mr \cdot Vsol \cdot Vcuv / Ebr \cdot Vsample,$$

where:

D is the optical density of the solution at a wavelength of 570 nm;

Mr is the molecular weight of bacteriorhodopsin (26,700);

Vsol is the total volume of the bacteriorhodopsin solution;

Vcuv is the volume of a solution of bacteriorhodopsin in a spectrophotometric cuvette;

Ebr is the molar absorption coefficient of bacteriorhodopsin (63000 M-1cm-1);

Vsample is the volume of a sample of bacteriorhodopsin in a spectrophotometric cuvette.

EXAMPLE 1. The use of a cosmetic agent to maintain the normal functioning of the facial and body skin.

An aqueous solution of bacteriorhodopsin is used at a concentration of 2.4 mg/ml (0.24%).

Patients aged 23-47 applied the product to dry clean skin of the face and body. The proposed cosmetic product is applied once and daily. The effect of the application is achieved on days 2-3. In the future, after reaching the desired effect, the proposed remedy is used at the discretion of the patient. Some patients treated the face with a tonic before applying the bacteriorhodopsin solution.

All patients noted that the proposed remedy for facial and body skin care provides an improvement in the appearance of the skin. The skin acquires smoothness, softness, elasticity, wrinkles are smoothed, the skin color acquires a fresh, healthy appearance. No adverse effects were observed for the entire duration of the application of the proposed agent.

EXAMPLE 2. The use of a cosmetic agent for psoriasis treatment.

An aqueous solution of bacteriorhodopsin is used at a concentration of 2.4 mg/ml (0.24%).

Patients aged 33-57 applied the product to the affected area of the skin. The proposed cosmetic product is applied once and daily. The effect of the application is achieved on days 4-7. Later, after reaching the desired effect, recurrence of the disease was not observed.

All patients noted that the proposed remedy eliminates the affected areas on the skin and provides an improvement in the appearance of the skin. The skin acquires a natural healthy appearance. No adverse effects were observed for the entire duration of the application of the proposed agent. No side effects were observed during the application of the recommended agent.

EXAMPLE 3. The use of a cosmetic agent for neurodermatitis treatment.

An aqueous solution of bacteriorhodopsin is used at a concentration of 2.4 mg/ml (0.24%) or 7.5 mg/ml (0.75%).

Patients aged 30-67 applied the product to the affected area of the skin. The proposed cosmetic product was applied daily in the morning and in the evening. In the case of exacerbation of the neurodermatitis within a period not exceeding 4 months, a solution of bacteriorhodopsin at a concentration of 2.4 mg/ml (0.24%) was used. In the first day (up to 5 days) of the use of the product, a decrease and disappearance of the itching, a decrease of affected areas and a decrease in the intensity of inflammation of the cutaneous lesions were observed. The maximum effect of the proposed solution was observed in 3 weeks. No side effects were observed during the application of the recommended agent.

During the course of neurodermatitis in chronic and prolonged stages, a bacteriorhodopsin solution of 7.5 mg/ml (0.75%) is used. In this case, the proposed cosmetic product was also applied daily in the morning and in the evening. The clinical picture of the effect of using the recommended preparation was the same as described for a concentration of 2.4 mg/ml (0.24%). No side effects were observed during the application of the recommended agent.

EXAMPLE 4. The use of a cosmetic agent for atopic dermatitis treatment.

An aqueous solution of bacteriorhodopsin is used at a concentration of 2.4 mg/ml (0.24%) and 7.5 mg/ml (0.75%) was used.

Patients aged 18-23 applied the product to the affected area of the skin. The proposed cosmetic remedy was applied daily in the morning and in the evening. In case of exacerbation of atopic dermatitis within a period not exceeding 4 months, a solution of bacteriorhodopsin at a concentration of 2.4 mg/ml (0.24%) was used. In the first day (up to 5 days) of the use, a decrease and disappearance of the itching, a decrease of affected areas and a decrease in the intensity of inflammation of the cutaneous lesions were observed. The maximum effect of the proposed solution was observed in 3 weeks. No side effects were observed during the application of the recommended agent. Some patients used an additional moisturizer and applied it after a solution of bacteriorhodopsin.

During the course of atopic dermatitis in chronic and prolonged stages, a solution of bacteriorhodopsin at a concentration of 7.5 mg/ml (0.75%) is used. In this case, the proposed cosmetic product was also applied daily in the morning and in the evening. The clinical picture of the effect of using the recommended preparation was the same as that described for a concentration of 2.4 mg/ml (0.24%). No side effects were observed during the application of the recommended agent.

**Claims**

1. Use of an aqueous solution of bacteriorhodopsin of the strain of the halophilic bacteria Halobacterium salinarum VKPM V-11850 containing bacteriorhodopsin at a concentration of 2.4 mg/ml (0.24%) — 7.5mg/ml (0.75%) as an active component of a cosmetic skin care product, wherein the bacteriorhodopsin is obtained as follows:

   - growing for 6 days the strain of halophilic bacteria Halobacterium salinarum VKPM V-11850 in 750 ml flasks in a volume of a growing medium of 300 ml under fluorescent lighting until the stationary growth stage on a circular shaker at 37°C and shaking 100 rpm, wherein for cultivation, the medium of the following composition is used (mass%): peptone - 1, yeast extract - 0.5, NaCl - 25, $MgSO_4$ - 2, KCl - 0.2, sodium citrate - 0.3, glycerin - 0.1, CaCl2 - 0.02, water - the rest, pH of the environment - 7.2-7.4;
   - after 6 days, separating the precipitate by centrifugation at 7000 g for 10 minutes and weighing the obtained biomass;
   - adding distilled water in a volume of 300 ml to 20 g of the separated precipitate and incubating the separated precipitate in the distilled water with stirring at room temperature for 16 hours;
   - after centrifugation at 7000 g for 10 min and 4°C, discarding the precipitate;
   - centrifuging at 50,000 g the resultant supernatant for 40 min and 4°C;
   - discarding the supernatant;
   - pouring distilled water in the same volume into the obtained precipitate;
   - suspending the precipitate and centrifuging the resulting bacterial lysate suspension at 50,000 g for 40 minutes and 4°C and repeating the manipulation of suspending the precipitate in water followed by high-speed centrifugation until the ratio of the optical density of the solution at a wavelength 280 nm to the optical density of the solution at a wavelength of 570 nm (D280\D570) is less than 2.5 in the suspended precipitate after the next centrifugation; and
- diluting the supernatant with distilled water to a concentration of bacteriorhodopsin of 2.4 mg/ml (0.24%) - 7.5mg/ml (0.75%).

2. A therapeutic agent for use in the complex treatment of dermatoses comprising an aqueous solution of bacteriorhodopsin of the strain of the halophilic bacteria Halobacterium salinarum VKPM V-11850 containing bacteriorhodopsin at a concentration of 2.4 mg/ml (0.24%) — 7.5mg/ml (0.75%) , wherein the bacteriorhodopsin is obtained as follows:

- growing for 6 days the strain of halophilic bacteria Halobacterium salinarum VKPM V-11850 in 750 ml flasks in a volume of a growing medium of 300 ml under fluorescent lighting until the stationary growth stage on a circular shaker at 37°C and shaking 100 rpm, wherein for cultivation, the medium of the following composition is used (mass%): peptone - 1, yeast extract - 0.5, NaCl - 25, $MgSO_4$ - 2, KCl - 0.2, sodium citrate - 0.3, glycerin - 0.1, CaCl2 - 0.02, water - the rest, pH of the environment - 7.2-7.4;
- after 6 days, separating the precipitate by centrifugation at 7000 g for 10 minutes and weighing the obtained biomass;
- adding distilled water in a volume of 300 ml to 20 g of the separated precipitate and incubating the separated precipitate in the distilled water with stirring at room temperature for 16 hours;
- after centrifugation at 7000 g for 10 min and 4°C, discarding the precipitate;
- centrifuging at 50,000 g the resultant supernatant for 40 min and 4°C;
- discarding the supernatant;
- pouring distilled water in the same volume into the obtained precipitate;
- suspending the precipitate and centrifuging the resulting bacterial lysate suspension at 50,000 g for 40 minutes and 4°C and repeating the manipulation of suspending the precipitate in water followed by high-speed centrifugation until the ratio of the optical density of the solution at a wavelength 280 nm to the optical density of the solution at a wavelength of 570 nm (D280\D570) is less than 2.5 in the suspended precipitate after the next centrifugation; and
- diluting the supernatant with distilled water to a concentration of bacteriorhodopsin of 2.4 mg/ml (0.24%) - 7.5mg/ml (0.75%).

**Patentansprüche**

1. Verwenden einer wässrigen Lösung von Bacteriorhodopsin des Stammes des halophilen Bakteriums Halobacterium salinarum VKPM V-11850, die Bacteriorhodopsin in einer Konzentration von 2,4 mg/ml (0,24%) - 7,5mg/ml (0,75%) enthält, als aktive Komponente eines kosmetischen Hautpflegeprodukts, wobei das Bacteriorhodopsin wie folgt erhalten wird:

- Züchten des Stammes des halophilen Bakteriums Halobacterium salinarum VKPM V-11850 für 6 Tage in 750-ml-Flaschen in einem Volumen eines Wachstumsmediums von 300 ml unter Fluoreszenzbeleuchtung bis zum stationären Wachstumsstadium auf einem kreisförmigen Schüttler bei 37°C und Schütteln mit 100 U/min, wobei für die Kultivierung das Medium mit der folgenden Zusammensetzung verwendet wird (Masse-%): Pepton - 1, Hefeextrakt - 0. 5, NaCl - 25, $MgSO_4$ - 2, KCl - 0,2, Natriumcitrat - 0,3, Glycerin - 0,1, CaCl2 - 0,02, Wasser - der Rest, pH-Wert der Umgebung - 7,2-7,4;
- nach 6 Tagen Abtrennen des Niederschlags durch Zentrifugation bei 7000 g für 10 Minuten und Wiegen der erhaltenen Biomasse;
- Zugeben von 300 ml destilliertem Wasser zu 20 g des abgetrennten Niederschlags und Inkubieren des abgetrennten Niederschlags in dem destillierten Wasser unter Rühren bei Raumtemperatur für 16 Stunden;
- nach Zentrifugation bei 7000 g für 10 min und 4°C, Verwerfen des Niederschlags;
- Zentrifugieren des entstandenen Überstandes bei 50.000 g für 40 min und 4°C;
- Verwerfen des Überstandes;
- Aufgießen des erhaltenen Niederschlags mit destilliertem Wasser im gleichen Volumen;
- Suspendieren des Präzipitats und Zentrifugieren der resultierenden Bakterienlysatsuspension bei 50.000 g für 40 Minuten und 4°C und Wiederholen der Handhabung des Suspendierens des Präzipitats in Wasser, gefolgt von Hochgeschwindigkeitszentrifugation, bis das Verhältnis der optischen Dichte der Lösung bei einer Wellenlänge von 280 nm zu der optischen Dichte der Lösung bei einer Wellenlänge von 570 nm (D280\D570) weniger als 2,5 in dem suspendierten Präzipitat nach der nächsten Zentrifugation beträgt; und
- Verdünnen des Überstands mit destilliertem Wasser auf eine Konzentration von Bacteriorhodopsin von 2,4 mg/ml (0,24%) - 7,5mg/ml (0,75%).

2. Therapeutisches Mittel zur komplexen Behandlung von Dermatosen, umfassend eine wässrige Lösung von Bacteriorhodopsin des Stammes des halophilen Bakteriums Halobacterium salinarum VKPM V-11850, enthaltend Bacteriorhodopsin in einer Konzentration von 2,4 mg/ml (0,24%) - 7,5mg/ml (0,75%), wobei das Bacteriorhodopsin wie folgt erhalten wird:

- Züchten des Stammes des halophilen Bakteriums Halobacterium salinarum VKPM V-11850 für 6 Tage in 750-ml-Kolben in einem Volumen eines Wachstumsmediums von 300 ml unter fluoreszierender Beleuchtung bis zum stationären Wachstumsstadium auf einem kreisförmigen Schüttler bei 37°C und Schütteln mit 100 U/min, wobei für die Kultivierung das Medium mit der folgenden Zusammensetzung verwendet wird (Masse-%): Pepton - 1, Hefeextrakt - 0. 5, NaCl - 25, MgSO$_4$ - 2, KCl - 0,2, Natriumcitrat - 0,3, Glycerin - 0,1, CaCl2 - 0,02, Wasser - der Rest, pH-Wert der Umgebung - 7,2-7,4;
- nach 6 Tagen Abtrennen des Niederschlags durch Zentrifugation bei 7000 g für 10 Minuten und Wiegen der erhaltenen Biomasse;
- Zugeben von 300 ml destilliertem Wasser zu 20 g des abgetrennten Niederschlags und Inkubieren des abgetrennten Niederschlags in dem destillierten Wasser unter Rühren bei Raumtemperatur für 16 Stunden;
- nach Zentrifugation bei 7000 g für 10 min und 4°C, Verwerfen des Niederschlags;
- Zentrifugieren des entstandenen Überstandes bei 50.000 g für 40 min und 4°C;
- Verwerfen des Überstandes;
- Aufgießen des erhaltenen Niederschlags mit destilliertem Wasser im gleichen Volumen;
- Suspendieren des Präzipitats und Zentrifugieren der resultierenden Bakterienlysatsuspension bei 50.000 g für 40 Minuten und 4°C und Wiederholen der Handhabung des Suspendierens des Präzipitats in Wasser, gefolgt von Hochgeschwindigkeitszentrifugation, bis das Verhältnis der optischen Dichte der Lösung bei einer Wellenlänge von 280 nm zu der optischen Dichte der Lösung bei einer Wellenlänge von 570 nm (D280\D570) weniger als 2,5 in dem suspendierten Präzipitat nach der nächsten Zentrifugation beträgt; und
- Verdünnen des Überstands mit destilliertem Wasser auf eine Konzentration von Bakteriorhodopsin von 2,4 mg/ml (0,24%) - 7,5mg/ml (0,75%).

**Revendications**

1. Utilisation d'une solution aqueuse de bactériorhodopsine de la souche de la bactérie halophile Halobacterium salinarum VKPM V-11850 contenant de la bactériorhodopsine à une concentration de 2,4 mg/ml (0,24%) - 7,5mg/ml (0,75%) comme composant actif d'un produit cosmétique de soin de la peau, dans laquelle la bactériorhodopsine est obtenue comme suit :

- culture pendant 6 jours de la souche de bactéries halophiles Halobacterium salinarum VKPM V-11850 dans des flacons de 750 ml dans un volume de milieu de culture de 300 ml sous éclairage fluorescent jusqu'au stade de croissance stationnaire sur un agitateur circulaire à 37°C et agitation 100 rpm, dans lequel pour la culture, le milieu de la composition suivante est utilisé (% en masse) : peptone - 1, extrait de levure - 0. 5, NaCl - 25, MgSO$_4$ - 2, KCl - 0,2, citrate de sodium - 0,3, glycérine - 0,1, CaCl2 - 0,02, eau - le reste, pH du milieu - 7,2-7,4 ;
- après 6 jours, séparer le précipité par centrifugation à 7000 g pendant 10 minutes et peser la biomasse obtenue ;
- ajouter de l'eau distillée dans un volume de 300 ml à 20 g du précipité séparé et incuber le précipité séparé dans l'eau distillée sous agitation à température ambiante pendant 16 heures ;
- après centrifugation à 7000 g pendant 10 min et à 4°C, rejeter le précipité ;
- centrifuger à 50 000 g le surnageant résultant pendant 40 min et à 4°C ;
- éliminer le surnageant ;
- verser de l'eau distillée dans le même volume dans le précipité obtenu ;
- mettre en suspension le précipité et centrifuger la suspension de lysat bactérien obtenue à 50 000 g pendant 40 minutes et 4°C et répéter la manipulation de mise en suspension du précipité dans l'eau suivie d'une centrifugation à grande vitesse jusqu'à ce que le rapport de la densité optique de la solution à une longueur d'onde de 280 nm à la densité optique de la solution à une longueur d'onde de 570 nm (D280\D570) soit inférieur à 2,5 dans le précipité en suspension après la centrifugation suivante ; et
- diluer le surnageant avec de l'eau distillée jusqu'à une concentration de bactériorhodopsine de 2,4 mg/ml (0,24%) - 7,5mg/ml (0,75%).

2. Agent thérapeutique pour le traitement complexe de dermatoses comprenant une solution aqueuse de bactériorhodopsine de la souche de la bactérie halophile Halobacterium salinarum VKPM V-11850 contenant de la bactériorhodopsine à une concentration de 2,4 mg/ml (0,24%) - 7,5 mg/ml (0,75%), dans lequel la bactériorhodopsine est obtenue comme suit :

- culture pendant 6 jours de la souche de bactéries halophiles Halobacterium salinarum VKPM V-11850 dans des flacons de 750 ml dans un volume de milieu de culture de 300 ml sous éclairage fluorescent jusqu'au stade de croissance stationnaire sur un agitateur circulaire à 37°C et agitation 100 rpm, dans lequel pour la culture, le milieu de la composition suivante est utilisé (% en masse) : peptone - 1, extrait de levure - 0. 5, NaCl - 25, $MgSO_4$ - 2, KCl - 0,2, citrate de sodium - 0,3, glycérine - 0,1, CaCl2 - 0,02, eau - le reste, pH du milieu - 7,2-7,4 ;

- après 6 jours, séparer le précipité par centrifugation à 7000 g pendant 10 minutes et peser la biomasse obtenue ;

- ajouter de l'eau distillée dans un volume de 300 ml à 20 g du précipité séparé et incuber le précipité séparé dans l'eau distillée sous agitation à température ambiante pendant 16 heures ;

- après centrifugation à 7000 g pendant 10 min et à 4°C, rejeter le précipité ;

- centrifuger à 50 000 g le surnageant résultant pendant 40 min et à 4°C ;

- éliminer le surnageant ;

- verser de l'eau distillée dans le même volume dans le précipité obtenu ;

- mettre en suspension le précipité et centrifuger la suspension de lysat bactérien obtenue à 50 000 g pendant 40 minutes et 4°C et répéter la manipulation de mise en suspension du précipité dans l'eau suivie d'une centrifugation à grande vitesse jusqu'à ce que le rapport de la densité optique de la solution à une longueur d'onde de 280 nm à la densité optique de la solution à une longueur d'onde de 570 nm (D280\D570) soit inférieur à 2,5 dans le précipité en suspension après la centrifugation suivante ; et

- diluer le surnageant avec de l'eau distillée jusqu'à une concentration de bactériorhodopsine de 2,4 mg/ml (0,24%) - 7,5mg/ml (0,75%).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2109515

**Non-patent literature cited in the description**

- **CAVALIER-SMITH T.** The neomuran origin of archaebacteria, the negibacterial root of the universal tree and bacterial megaclassification. *Int. J. of Syst. And Envir.Microb.,* 2002, vol. 52, 7-76